(19) **Europäisches Patentamt / European Patent Office / Office européen des brevets**

(11) **EP 3 803 885 B1**

(12) **FASCICULE DE BREVET EUROPEEN**

(45) Date de publication et mention
de la délivrance du brevet:
**10.09.2025 Bulletin 2025/37**

(21) Numéro de dépôt: **19740626.7**

(22) Date de dépôt: **28.05.2019**

(51) Classification Internationale des Brevets (IPC):
**G16C 20/30** *(2019.01)*

(52) Classification Coopérative des Brevets (CPC):
**G16C 20/30**

(86) Numéro de dépôt international:
**PCT/FR2019/051259**

(87) Numéro de publication internationale:
**WO 2019/229378 (05.12.2019 Gazette 2019/49)**

(54) **DETERMINATION PREDICTIVE DE POTENTIELS CHIMIQUES D'EXCES**

PRÄDIKTIVE BESTIMMUNG VON ÜBERSCHÜSSIGEN CHEMISCHEN POTENZIALEN

PREDICTIVE DETERMINATION OF EXCESS CHEMICAL POTENTIALS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **28.05.2018 FR 1854525**

(43) Date de publication de la demande:
**14.04.2021 Bulletin 2021/15**

(73) Titulaire: **ROQUETTE FRERES
62136 Lestrem (FR)**

(72) Inventeurs:
• **TOURE, Oumar
62400 Bethune (FR)**
• **BOIT, Baptiste
59253 La Gorgue (FR)**
• **DUSSAP, Claude-Gilles
63000 Clermont Ferrand (FR)**

(74) Mandataire: **Plasseraud IP
104 Rue de Richelieu
CS92104
75080 Paris Cedex 02 (FR)**

(56) Documents cités:
• **POHORILLE A. ET AL: "Excess chemical potential of small solutes across water-membrane and water-hexane interfaces", JOURNAL OF CHEMICAL PHYSICS, AMERICAN INSTITUTE OF PHYSICS, US, vol. 104, no. 10, 1 January 1996 (1996-01-01), pages 3760 - 3773, XP009511120, ISSN: 0021-9606, DOI: 10.1063/1.471030**
• **BOULOUGOURIS G. C. ET AL: "On the calculation of the chemical potential using the particle deletion scheme", MOLECULAR PHYSICS, vol. 96, no. 6, 20 March 1999 (1999-03-20), GB, pages 905 - 913, XP055557135, ISSN: 0026-8976, DOI: 10.1080/00268979909483030**
• **HESS B. ET AL: "Fast-Growth Thermodynamic Integration: Calculating Excess Chemical Potentials of Additive Molecules in Polymer Microstructures", MACROMOLECULES, vol. 41, no. 6, 1 March 2008 (2008-03-01), US, pages 2283 - 2289, XP055555792, ISSN: 0024-9297, DOI: 10.1021/ma702070n**
• **GAIDA L. B. ET AL: "Variable hydration of small carbohydrates for predicting equilibrium properties in diluted and concentrated solutions", FOOD CHEMISTRY, vol. 96, no. 3, 1 June 2006 (2006-06-01), NL, pages 387 - 401, XP055555791, ISSN: 0308-8146, DOI: 10.1016/j.foodchem.2005.02.053**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- DE P. SOARES R. ET AL: "The Combinatorial Term for COSMO-Based Activity Coefficient Models", INDUSTRIAL & ENGINEERING CHEMISTRY RESEARCH, vol. 50, no. 5, 2 March 2011 (2011-03-02), pages 3060 - 3063, XP055556334, ISSN: 0888-5885, DOI: 10.1021/ie102087p
- SHAH P. P. ET AL: "Molecular Solvation in Water-Methanol and Water-Sorbitol Mixtures: The Roles of Preferential Hydration, Hydrophobicity, and the Equation of State", JOURNAL OF PHYSICAL CHEMISTRY PART B, vol. 111, no. 17, 1 May 2007 (2007-05-01), US, pages 4467 - 4476, XP055556529, ISSN: 1520-6106, DOI: 10.1021/jp0688714
- CATTÉ M. ET AL: "A physical chemical UNIFAC model for aqueous solutions of sugars", FLUID PHASE EQUILIBRIA, vol. 105, no. 1, 1 March 1995 (1995-03-01), AMSTERDAM, NL, pages 1 - 25, XP055556536, ISSN: 0378-3812, DOI: 10.1016/0378-3812(94)02604-Y
- LEE S. ET AL: "Excess Gibbs Potential Model for Multicomponent Hydrogen Clathrates", JOURNAL OF PHYSICAL CHEMISTRY PART B: CONDENSED MATTER, MATERIALS, SURFACES, INTERFACES & BIOPHYSICAL, vol. 110, no. 51, 1 December 2006 (2006-12-01), US, pages 26122 - 26128, XP055556535, ISSN: 1520-6106, DOI: 10.1021/jp063431y

## Description

L'invention concerne le domaine de la détermination prédictive des propriétés physico-chimiques des solutions.

La détermination prédictive des propriétés physico-chimiques d'une solution présente un intérêt industriel très important. En effet, une installation industrielle étant amenée à traiter des volumes importants, il est important de maitriser au mieux les différents paramètres d'un processus de fabrication mettant en œuvre une solution, afin de s'assurer de la bonne adéquation de l'installation avec le calibrage du processus. Par exemple, certaines installations ne pourront pas gérer des volumes trop importants, d'autres ne pourront gérer que des liquides et il conviendra donc de s'assurer qu'aucun phénomène d'ébullition n'intervient, d'autres encore pourront gérer des gaz mais seulement dans une gamme de pression donnée au-delà de laquelle des problèmes de sécurité surgiront. De même il peut être intéressant de connaître précisément la quantité d'énergie calorifique à fournir à un produit en vue de sa cuisson ou de sa transformation afin d'éviter d'autres réactions parasites et une surconsommation d'énergie.

La détermination des paramètres et le calibrage des installations industrielles revêt donc une importance majeure. Bien qu'il soit parfois possible d'effectuer ce calibrage par essai-erreur, cette méthode n'est pas toujours satisfaisante car elle ne permet pas de s'assurer que l'on est dans des conditions optimales. Dès lors, l'utilisation de méthodes de détermination prédictives, par exemple mises en œuvre par des logiciels de simulation, sont particulièrement intéressantes. Ces méthodes de détermination prédictives permettent de modéliser les coefficients d'activité ou les potentiels chimiques de molécules.

A ce titre, il est connu des documents WO2015175387 et WO2012051242 de modéliser le coefficient d'activité d'une molécule dans une solution d'intérêt en approchant chaque molécule grâce à un algorithme empirique de moyennage du profil de densité de charge (ou histogramme moléculaire des densités de charge), similaires à la méthode décrite dans l'article Klamt et al., J. Phys. Chem. A, 1998, Volume 102, n°26, pages 5074-5085, cette méthode étant connue sous la dénomination de « méthode de sigma-averaging ». Ces modèles utilisent une approche purement physique et combinatoire, ce qui peut être satisfaisant dans le cas de certaines solutions aqueuses simples et diluées mais qui donne des résultats très éloignés de la réalité lorsque l'on cherche à modéliser des solutions concentrées au sein desquelles des équilibres chimiques sont en place, notamment des équilibres d'associations chimiques (complexation, solvatation, hydratation,...) déduites des propriétés de liaisons entre les molécules composant la solution. En effet, ces modèles ne permettent pas de rendre compte des interactions chimiques entre les différentes molécules. C'est notamment le cas des solutions aqueuses concentrées de sucres et/ou de polyols et/ou de carbohydrates.

Or, dans un contexte industriel, les composants intéressants au sein d'une solution sont généralement les solutés et pas le solvant et l'approche purement physique et combinatoire est rarement adaptée à ces solutions aqueuses concentrées.

Il existe donc un besoin pour une méthode de détermination prédictive de grandeurs physico-chimiques d'équilibres dans un contexte industriel, en particulier dans le cas de solutions aqueuses concentrées ou présentant un taux de matière sèche très important. En effet, des tentatives de modélisation des solutions aqueuses ont déjà été réalisées. Dans cet objectif, Catté et al. (Fluid Phase Equilibria 105, 1-25., 1995) ont combiné le modèle physique UNIFAC avec un modèle chimique d'hydratation fixe des sucres par l'eau en vue de modéliser des solutions aqueuses plutôt diluées. Toutefois, la Demanderesse a pu constater que ce type de modèle ne fonctionne pas pour des solutions aqueuses concentrées.

L'invention y parvient grâce à une méthode de détermination prédictive d'un potentiel chimique d'excès d'une espèce apparente comportant les étapes de la revendication 1.

Par espèce vraie, on entend une entité chimique présente en solution et pas toujours isolable. Par exemple, dans le cas d'une solution aqueuse de sorbitol, on distinguera le sorbitol isolé, le sorbitol associé à une molécule d'eau, le sorbitol associé à deux molécules d'eau et le sorbitol associé à trois molécules d'eau comme autant d'espèces vraies.

Par espèce apparente, on entend les espèces en solution isolables et dont on pourra mesurer facilement les propriétés. Dans le cas d'une solution aqueuse de sorbitol, il s'agit par exemple de l'eau d'une part, et du sorbitol d'autre part.

Les notions d'espèces vraies (en anglais « true species ») et apparentes (en anglais « apparent species ») sont connues en elles-mêmes. Par exemple, Prausnitz et al. les a déjà décrites dans l'ouvrage de référence Molecular thermodynamics of fluid-phase equilibria, Prentice-Hall international series in the physical and chemical engineering sciences, Prentice-Hall PTR., 1999, page 353. Elles apparaissent également dans différentes publications telles que celles de Toure et al. (The Canadian Journal Of Chemical Engineering 93, 2015, page 445 ainsi que Fluid Phase Equilibria, 2016, 424, page 92). Achard et al., (AIChE journal, 40(7), 1994. 1210-1222) et la publication de Catté et al. précédemment citée font également référence aux propriétés respectives des espèces vraies quand ils parlent de « concentration ou fraction molaire vraie », et des espèces apparentes en parlant de « concentration ou fraction molaire apparente ». Alternativement, une espèce vraie peut également être appelée entité moléculaire et espèce apparente peut être appelée espèce chimique (ou ensemble d'entités moléculaires) tels que définis par l'IUPAC.

Le potentiel chimique d'excès est déterminé à partir d'une propriété molaire partielle, conduisant après comparaison entre différentes solutions aux potentiels chimiques.

De préférence, la méthode de détermination prédictive d'un potentiel chimique d'excès selon l'invention comporte en outre une étape consistant à effectuer un pavage de façon à approcher chaque espèce vraie déterminée à l'étape a par une combinaison de solides de Platon de sorte qu'elle soit associée à une forme présentant une surface extérieure constituée d'une combinaison de segments surfaciques plans et de même surface.

La détermination d'un potentiel chimique d'excès, également appelé enthalpie libre d'excès, est particulièrement intéressante car elle permet de déterminer la plupart des propriétés physico-chimiques de la solution. En effet, le potentiel chimique d'excès de la solution contribue à rendre notamment compte de la disponibilité des molécules.

Par espèce chimique, on n'entend pas se limiter à une molécule donnée. Une même molécule peut correspondre à plusieurs espèces chimiques différentes selon qu'elle est en interaction ou pas avec une ou plusieurs autres molécules. Grâce au fait que la typologie se fait par espèce chimique, et non par molécule, l'étape consistant à effectuer une typologie des espèces chimiques en présence permet de rendre compte des interactions au sein de la solution. Lors de cette étape, on peut également prendre en compte la probabilité de présence d'une espèce donnée, de façon à rendre compte de divers équilibres physico-chimiques. En d'autres termes, la « molécule » est une « espèce apparente » présente dans le mélange et l'« espèce chimique » est une « espèce vraie » réellement présente dans le mélange. Par exemple, la molécule pourrait être le *sorbitol* et les *espèces chimiques* correspondantes seraient les différents états d'hydratation du sorbitol.

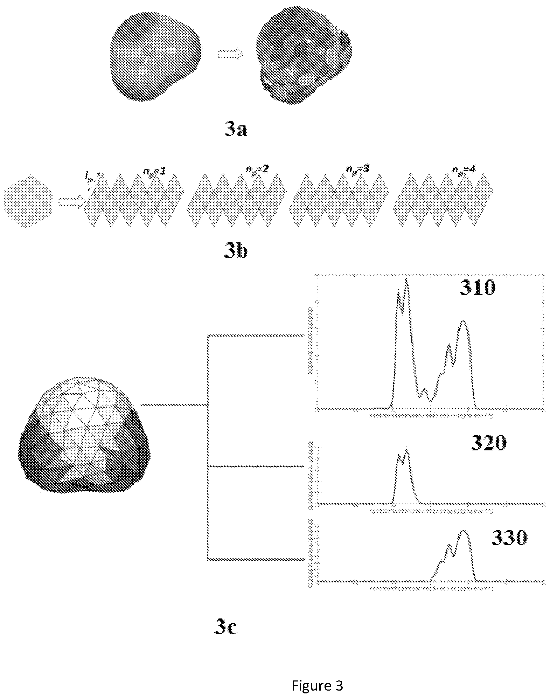

Après avoir déterminé la typologie des différentes espèces vraies en présence, il est nécessaire de générer préalablement un certain nombre de données d'entrée. Ces données d'entrée sont extraites d'un résultat de calcul quantique, par exemple sous la forme d'un fichier COSMO pour chaque espèce chimique. Un exemple de visualisation du contenu d'un fichier COSMO est illustré sur la figure 3a où la couleur est une représentation de la valeur du contenu du vecteur de grandeur physique associé à une espèce vraie. Pour rappel, un fichier COSMO comprend, pour chacun des segments formant la surface externe de l'espèce chimique, le potentiel électrostatique, la surface, la charge et la densité surfacique de charge.

De préférence, et en particulier lorsque le vecteur de grandeurs physiques comprend des densités surfaciques de charge, chaque espèce chimique identifiée est alors approchée par une combinaison de solides de Platon identiques. Un solide de Platon est un polyèdre régulier et convexe ayant des surfaces externes identiques sur chacune de ses faces, ce qui lui permet de paver sans discontinuité un espace à trois dimensions.

L'utilisation de solides de Platon identiques constitue alors une innovation majeure. En effet, en sortie de calcul quantique, les segments formant la surface externe d'une molécule présentent des tailles différentes ; les méthodes usuelles de modélisation de coefficient d'activité (notamment celles des modèles évoqués plus haut) consistent à moyenner de façon empirique les densités de charge surfaciques desdits segments par une surface plane identique (généralement carrée ou circulaire) de taille adaptée. En utilisant des solides de Platon identiques, tout l'espace représentant le volume de la solution est pavé de façon compacte et chaque molécule présente une surface externe consistant en une combinaison de surfaces identiques, de véritables segments surfaciques, ce qui permet de comparer les molécules entre elles et de modéliser les interactions entre différents segments pour pouvoir combiner de façon cohérente un modèle physique avec un modèle chimique. Ceci permet notamment de pouvoir modéliser les propriétés physico-chimiques des solutions aqueuses concentrées sus-mentionnées.

A cet effet, on associe à chaque espèce vraie déterminée au moins un vecteur de grandeurs physiques. Si un pavage en solides de Platon a été utilisé, on génère de préférence un vecteur de grandeurs physiques par segment surfacique. Le terme vecteur s'entend ici dans son acception algébrique : il s'agit d'un tableau regroupant un certain nombre de grandeurs physiques relatives au segment surfacique auquel il est associé.

Lorsque chaque vecteur se rattache à un segment surfacique de même dimension, chaque vecteur a le même poids.

L'ensemble des vecteurs constitue donc un bon descriptif de la solution à partir duquel il est possible de prédire un potentiel chimique d'excès d'une espèce apparente, ce qui est particulièrement utile dans le cadre d'une installation industrielle car cela permet de contrôler l'adéquation de la méthode prédictive avec la réalité au moyen d'une mesure.

La détermination des potentiels chimiques d'excès respectifs des espèces vraies se fait en utilisant une vision physique des interactions entre molécules et/ou entre espèces chimiques. Un choix judicieux de la typologie des espèces vraies permet ainsi d'associer de façon cohérente à cette vision physique des interactions un modèle chimique d'associations chimiques, et donc d'effectuer une recomposition de la solution en espèces équivalentes pour construire la prédiction des potentiels chimiques d'excès respectifs des espèces apparentes.

La solution est de préférence une solution aqueuse de sucres et/ou de polyols et/ou de carbohydrates. En effet, le modèle selon l'invention est particulièrement adapté à ce type de solutions qui peuvent être très concentrées et présenter de nombreuses espèces chimiques différentes, notamment en raison des nombreuses molécules d'eau pouvant être adsorbées par les molécules.

Les carbohydrates incluent les sucres et les polyols. Les termes « sucres », « polyols » et « carbohydrates » sont bien connus de l'Homme du métier. Les polyols sont également connus sous le terme « alditols ». Pour chacun de ces termes, l'Homme du métier peut se référer aux définitions de l'International Union of Pure and Applied Chemistry (IUPAC) qui figurent dans l'article Moss et al., Pure Applied Chemistry, 1995, 67, 1307, Glossary of class names of organic compounds and reactivity intermediates based on structure (IUPAC Recommendations 1995).

Les solutions de carbohydrates peuvent notamment être obtenues à partir d'amidon. En ce qui concerne les solutions aqueuses de sucres, il peut s'agir d'une solution de maltodextrines, d'un sirop de glucose ou d'un sirop de fructose.

A titre d'exemples de sucres, on peut citer le glucose, l'arabinose, le xylose, le fructose, le psicose (encore appelé allulose), le mannose, le ribose, le galactose, le tréhalose, le cellobiose, le gentiobiose, l'isomaltose, l'isomaltulose, le kojibiose, le laminaribiose, le maltose, le galactose, le lactose, le maltulose, le nigerose, le saccharose et le sophorose.

Des carbohydrates autres que les sucres précités peuvent être des saccharides de degré de polymérisation supérieur ou égal à 3. Il peut s'agir d'oligosaccharides de degré de polymérisation supérieur ou égal à 3, notamment des oligosaccharides présentant un degré de polymérisation allant de 3 à 20 et notamment des oligosaccharides tels que le maltotriose, l'isomaltotriose, le panose, le raffinose, le maltotétratose et les cyclodextrines.De manière générale, les polyols peuvent être tout saccharide sus-mentionné hydrogéné. Ils peuvent par exemple être le polyglucitol, c'est-à-dire une solution de sirop de glucose ou de maltodextrine hydrogénée. Les polyols peuvent être des polyols comprenant de 3 à 24 atomes de carbone comme par exemple le glycérol, l'erythritol, le threitol, l'arabitol, le xylitol, le ribitol, le mannitol, le sorbitol, le galactitol, le fucitol, l'iditol, l'inositol, le volemitol, l'isomalt, le maltitol, le lactitol, le maltotriitol et le matotétraitol. De préférence, les polyols sont choisis parmi le sorbitol, le mannitol, le xylitol et le maltitol.

La combinaison de solides de Platon est par exemple une combinaison d'icosaèdres. L'icosaèdre présente vingt faces triangulaires. Il s'agit du type de solide de Platon présentant le plus grand nombre de faces et permettant donc d'obtenir un pavage approchant au mieux chaque espèce chimique en présence. Alternativement, le solide de Platon est un dodécaèdre constitué de douze faces de pentagones réguliers identiques.

De préférence, au moins un desdits vecteurs de grandeurs physiques contient une grandeur physique choisie parmi la densité de charges, la propension à former une liaison hydrogène en tant que donneur d'atome d'hydrogène, la propension à former une liaison hydrogène en tant qu'accepteur d'atome d'hydrogène, et le potentiel électrostatique. Au lieu de la densité de charge, il est évidemment également possible d'utiliser comme grandeur physique la charge et/ou la surface.

Ces grandeurs physiques permettent de décrire au mieux les interactions dans la solution et d'en dériver ses propriétés, et en particulier les différents potentiels chimiques pertinents, de façon précise. Selon les grandeurs physiques utilisées, les équations utilisées pour remonter au potentiel chimique ne seront pas les mêmes.

Selon le type de solution utilisé, les interactions inter-espèces chimiques peuvent être différentes et il peut donc être intéressant d'utiliser d'autres grandeurs physiques, plus adaptées.

La typologie des espèces chimiques en présence distingue par exemple différents états de complexation d'une même molécule.

De préférence, la typologie des espèces chimiques en présence distingue différents états de solvatation, en particulier d'hydratation, d'une même molécule. Il est entendu que la solvatation est un mode particulier de complexation par le solvant, l'hydratation étant une solvatation par de l'eau. Cette typologie des espèces chimiques permet ainsi d'associer à la vision physique susmentionnée de la détermination des potentiels chimiques d'excès des espèces chimiques, une contribution d'associations chimiques déduites des propriétés de liaisons entre les molécules composant la solution. De ce fait, les espèces chimiques réellement présentes dans le mélange ont des propriétés physiques différentes des molécules individuelles formant le mélange apparent. Par conséquent, une recomposition de la solution en espèces équivalentes permet de construire la prédiction des propriétés molaires partielles conduisant à la détermination du potentiel chimique d'excès des molécules (ou espèces apparentes).

La méthode de détermination prédictive d'un potentiel chimique d'excès selon l'invention peut comporter en outre une étape c' consistant à effectuer un histogramme moléculaire d'au moins une desdites grandeurs physiques.

Un tel histogramme permet un traitement statistique de la solution. Un sigma-profile est un exemple d'un tel histogramme, appliqué au profil de densité de charges d'une solution.

La méthode de détermination prédictive d'un potentiel chimique d'excès selon l'invention peut comporter en outre une étape consistant à déterminer une grandeur déduite du potentiel chimique d'excès des molécules (ou espèces apparentes). La connaissance du potentiel chimique d'excès permet de traduire l'écart à l'idéalité des solutions et de déduire l'ensemble des propriétés thermodynamiques (activité de l'eau $a_w$, coefficients d'activité $\gamma_i$ des constituants, coefficient osmotique) et d'équilibres entre phases (équilibres liquide-liquide, liquide-vapeur et liquide-solide), à partir des relations générales de la thermodynamique. Par exemple, l'équilibre liquide-vapeur est caractérisé par la notion de température d'ébullition du mélange. De préférence, cette grandeur déduite du potentiel chimique est choisie parmi un coefficient d'activité d'une espèce chimique ou d'une molécule en présence, une température d'ébullition de la solution, et une solubilité d'une espèce chimique ou d'une molécule en présence.

Le potentiel chimique d'excès de la solution permet en effet de retrouver un grand nombre de grandeurs d'intérêt de la solution, qui pourront servir le propos de telle ou telle installation asservie à la méthode selon l'invention.

La solution présente de préférence une teneur massique en solvant inférieure à 30% par rapport à la masse totale de la solution, avantageusement inférieure à 20%.

En effet, bien que la méthode de détermination prédictive selon l'invention soit adaptée à tout type de solution, la précision des résultats obtenus est particulièrement intéressante dans le domaine des solutions concentrées pour lesquelles il n'existe aucune alternative simple.

Les solides de Platon choisis présentent par exemple une dimension caractéristique comprise entre 0.0005 Å et 100Å, de préférence entre 0.0005 Å et 6 Å. La dimension caractéristique d'un solide de Platon correspond à la taille de son arête. Une dimension caractéristique de cet ordre permet un rendu suffisamment précis des propriétés de la solution.

L'invention a également pour objet l'utilisation d'une méthode selon l'invention afin de calibrer une installation industrielle. Tout type d'installation industrielle nécessite en effet un calibrage. Compte tenu des volumes mis en jeu au sein d'une installation industrielle, le fait de réaliser ce calibrage de façon prédictive, sans avoir à sacrifier une partie de la production à cet effet est particulièrement avantageux.

L'installation industrielle est de préférence une installation de production de sucres et/ou de polyols et/ou de carbohydrates. En effet, comme indiqué plus haut, la méthode selon l'invention est particulièrement adaptée aux solutions de sucres et/ou de polyols et/ou de carbohydrates plus généralement.

L'invention a également pour objet un système informatique comprenant un processeur, ledit processeur étant configuré pour mettre en œuvre une méthode selon l'invention lorsqu'il reçoit des instructions spécifiques.

L'invention a également pour objet un produit-programme d'ordinateur comprenant un code configuré pour réaliser une méthode selon l'invention lorsqu'il est exécuté par un processeur ou une unité de contrôle électronique.

L'invention pourra être mieux comprise à l'aide des exemples de réalisation non limitatifs décrits ci-après, et à l'examen du dessin annexé sur lequel :

- la figure 1 est une représentation de la structure tridimensionnelle du sorbitol et de ses différents états d'hydratation,

- la figure 2 est un diagramme représentant la structure globale et les données nécessaires à la mise en œuvre d'une méthode selon l'invention,

- la figure 3 représente différentes étapes de la génération d'un pavage de solides de Platon d'une molécule et des histogrammes de densité de charges correspondant,

- la figure 4 représente une comparaison des histogrammes moléculaires des densités de charge ($\sigma$-profile), pour le cas représentatif de la molécule d'eau, obtenus en utilisant la méthode susmentionnée de Klamt et al. et la méthode de l'invention,

- la figure 5 représente un diagramme schématisant une méthode de résolution permettant de calculer les différents potentiels chimiques d'excès d'une solution en mettant en œuvre la méthode selon l'invention,

- la figure 6 est une comparaison des données d'activité de l'eau obtenues en mettant en œuvre la méthode selon l'invention, en utilisant une méthode selon l'art antérieure et en les calculant à partir des températures d'ébullition à pression atmosphérique obtenues par mesure expérimentale,

- la figure 7 est une comparaison des données de température d'ébullition obtenues en utilisant le modèle thermodynamique développé dans cette invention, celles obtenues en utilisant les méthodes de l'état de l'art et en les obtenant par mesure expérimentale

- la figure 8 est une représentation schématique d'un évaporateur à couche mince à film raclé.

Les exemples qui suivent permettront de mieux appréhender la présente invention, sans pour autant en limiter la portée.

Afin d'effectuer une typologie des différentes espèces chimiques en présence, il convient de déterminer les différents équilibres physico-chimiques susceptibles d'intervenir au sein de la solution.

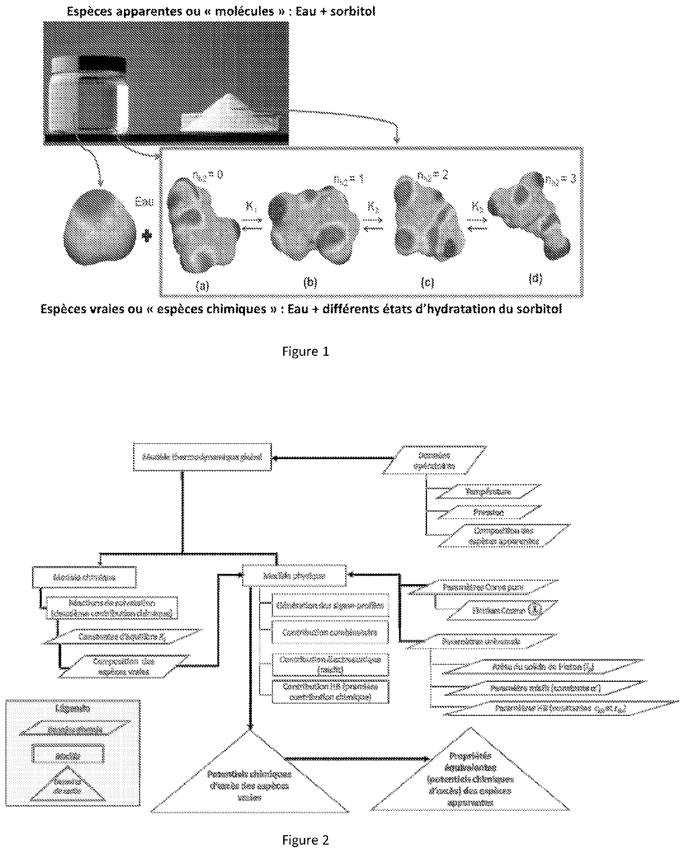

Dans le cas de cet exemple de réalisation non limitatif, on s'intéresse au cas du sorbitol. Le sorbitol est susceptible d'adsorber 0, 1, 2 ou 3 molécules d'eau selon la quantité d'eau disponible. Cinq espèces chimiques sont donc susceptibles d'être en présence ; ce sont les espèces illustrées à la figure 1.

Après avoir déterminé la typologie des différentes espèces vraies en présence, il est nécessaire de générer préalablement un certain nombre de données d'entrée. Ces données d'entrée sont extraites d'un résultat de calcul quantique, par exemple sous la forme d'un fichier COSMO pour chaque espèce chimique. Pour rappel, un fichier COSMO comprend, pour chacun des segments formant la surface externe de l'espèce chimique, la surface, la charge, la densité de charge et le potentiel électrostatique. Un exemple de visualisation du contenu d'un fichier COSMO est illustré sur la figure 3a où la couleur est une représentation de la valeur du contenu du vecteur de grandeur physique associé à une espèce vraie.

Ces fichiers COSMO font partie des données d'entrées du modèle physique comme illustré à la figure 2. De plus, des données opératoires (température, pression, composition des différentes molécules ou espèces apparentes) et des paramètres universels du modèle physique sont également utilisés pour déterminer les potentiels chimiques des espèces vraies en utilisant dans la partie chimique les constantes d'équilibre d'associations chimiques entre les molécules (solvatation) dans le modèle chimique. Une recomposition de la solution en espèces équivalentes permet ainsi de déterminer les potentiels chimiques d'excès des molécules (ou espèces apparentes) à partir de la connaissance des compositions vraies et des potentiels chimiques d'excès respectifs des espèces chimiques réellement présentes dans la solution. Pour ce faire, la surface moléculaire de chaque espèce chimique est préalablement pavée par une combinaison de solides de Platon de sorte qu'elle soit associée à une forme présentant une surface extérieure constituée d'une combinaison de segments surfaciques plans et de même surface, et un vecteur de grandeurs physiques est associé à chaque segment surfacique.

Un solide de Platon est un polyèdre régulier et convexe ayant des surfaces externes identiques sur chacune de ses faces.

De préférence, le solide de Platon est un dodécaèdre constitué de douze faces de pentagones réguliers identiques, tout préférentiellement un icosaèdre constitué de vingt faces triangulaires équilatérales identiques (Figure 3b). Ces solides se rapprochant plus de la surface d'une sphère, le pavage de la surface est plus proche de la valeur de la surface issue du calcul quantique et contenue dans le fichier COSMO (Figure 3a).

Préférentiellement, la valeur de la taille (lp) de l'arrête de ces solides de Platon est inférieure ou égale à 6 Å. Pour chaque espèce chimique, le sigma-profile moléculaire, c'est-à-dire un histogramme donnant le profil de densité de charge, 310 (p($\sigma$)) est généré en utilisant un pavage de la surface par un nombre entier de solides de Platon et en affectant à chaque face, ou segment surfacique, une valeur que l'on stocke dans un vecteur.

Cette génération permet de ne pas utiliser de méthode de sigma-averaging et donc de rester au plus proche des simulations quantiques.

La figure 3c illustre le sigma profile 310 obtenu.

Pour chaque segment surfacique, c'est-à-dire chaque face de solide de Platon, correspondant à une partie d'espèce chimique susceptible de réagir en tant que donneur ($\sigma$d) de liaison hydrogène (HB) d'une molécule, les sigma-profile donneurs 320 (pHB($\sigma$d)) sont générés, ces sigma-profiles étant générés en utilisant un pavage de leur surface par un nombre entier de solides de Platon, ce pavage étant préférentiellement identique au précédent.

De manière similaire, pour chaque segment accepteur ($\sigma$a) de liaison hydrogène (HB) d'une molécule, les sigma-profile accepteurs 330 (pHB($\sigma$a)) sont générés, ces sigma-profiles étant générés en utilisant un pavage de leur surface par un nombre entier de solides de Platon, ce pavage étant préférentiellement identique au précédent.

Chaque segment surfacique se voit donc attribué trois valeurs dans un vecteur correspondant à chacun des profils 310, 320, et 330.

Lesdits solides de Platon utilisés dans les différents pavages ont des arrêtes identiques et sont donc des solides de Platon de surface et volume identiques.

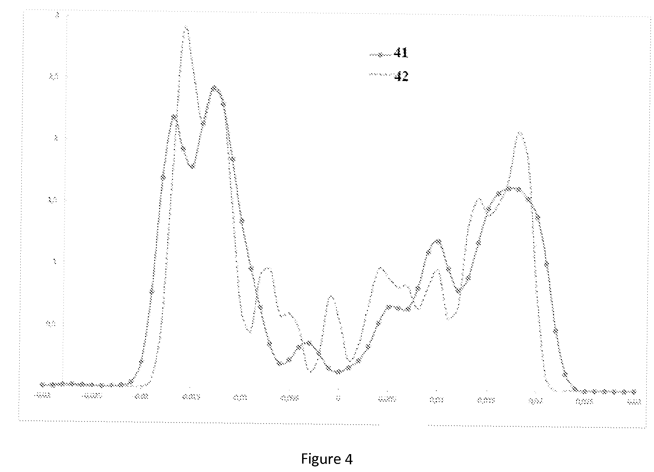

Comme représenté à la Figure 4, le sigma-profile 41 ainsi obtenu est différent de celui 42 obtenu par les méthodes de l'art antérieur utilisant le sigma-averaging. La figure 4 représente le sigma-profile moléculaire en fonction de la densité surfacique de charge en électron par Angstrom carré.

Le sigma-profile du mélange est la somme pondérée par les fractions molaires des sigma-profiles de chacune des molécules.

Dans la suite, le sigma-profile est donc utilisé comme descripteur de l'espèce chimique pavée.

Dans la partie physique de la méthode selon l'invention, comme illustré sur la figure 2, après la génération des sigma-profiles des espèces chimiques, l'étape d'obtention du potentiel chimique d'excès d'une molécule dans un mélange passe par l'obtention de trois différentes contributions.

La contribution combinatoire prend en compte les différences de taille et de forme entre les molécules présentes dans le mélange et rend compte de la probabilité que chaque face en rencontre une autre.

La contribution électrostatique, dite « contribution misfit » est générée de manière à prendre en considération le fait que les surfaces en interaction électrostatique sont des faces du solide de Platon.

La contribution dite « HB » est obtenue en considérant qu'elle résulte uniquement des interactions entre les parties donneurs et les parties accepteurs de liaison hydrogène (HB) contenues respectivement dans les sigma-profiles accepteur et donneur du mélange.

Contribution combinatoire

Pour prendre en compte les différences de taille et de forme entre les molécules présentes dans le mélange, la contribution combinatoire du potentiel chimique d'excès est calculée en utilisant la formule suivante :

$$\mu_i{}^{E,\,combi} = RT \cdot \ln\left(\gamma_i{}^{Combi}\right)\;;$$

où

$$\ln\left(\gamma_i^{Combi}\right) = \ln\left(\frac{\Phi_i}{x_i}\right) + \left(1 - \frac{\Phi_i}{x_i}\right) + \frac{q_i}{r_i}\cdot\ln\left(\frac{\theta_i}{\Phi_i}\right) + \frac{q_i}{r_i}\cdot\left(\frac{\Phi_i}{x_i} - \frac{\theta_i}{x_i}\right)$$

Cette formule est novatrice dans l'état de la technique. C'est une adaptation de la formule de type Staverman Guggenheim (SG) et permet de prendre en compte une contribution entropique qui tient compte des différences de volumes et de surface externes des molécules

$$\Phi_i = \frac{x_i \cdot r_i}{\sum_{j=1}^{n_{vrai}} x_j \cdot r_j}$$

$$\theta_i = \frac{x_i \cdot q_i}{\sum_{j=1}^{n_{vrai}} x_j \cdot q_j}$$

$$q_i = \frac{A_i}{S_{icosaèdre}}$$

$$r_i = \frac{V_i}{v_{icosaèdre}}$$

Contribution misfit

La contribution misfit du potentiel chimique d'excès résulte de la formule suivante :

$$\mu_i{}^{E,\,misfit} = \mu_i^{misfit,S} - \mu_i^{misfit,ref}$$

Où $\mu_i^{misfit,S}$ désigne la contribution misfit du potentiel chimique de la molécule dans la solution et $\mu_i^{misfit,ref}$ son potentiel chimique dans un état de référence choisi par l'utilisateur. Cet état de référence peut être choisi parmi l'état de référence corps purs pour chacun des constituants ou bien à l'état de référence dilution infinie dans le solvant majoritaire (e.g. eau) pour chacun des constituants, hormis le solvant majoritaire qui est dans un état de référence corps pur. $\mu_i^{misfit,S}$ est la somme, pondérée par les nombre des segments représentés dans le sigma-profile $p_i$ ($\sigma$), des coefficients d'activité des segments de surface constituant la molécule :

$$\mu_i^{misfit,S} = \int p_i(\sigma)\cdot\mu^{misfit,S}(\sigma)d\sigma$$

Le potentiel chimique du segment de surface $\mu^{misfit,S}(\sigma)$ dépend des énergies d'interaction électrostatiques entre tous les segments de surface.

$$\mu^{misfit,S}(\sigma) = -k \cdot T \cdot \ln\left(\int \overline{p^s(\sigma')} \cdot exp\left(\frac{\mu^{misfit,S}(\sigma') - E_{misfit}(\sigma,\sigma')}{k \cdot T}\right) d\sigma'\right)$$

Cette énergie d'interaction peut être calculée par la formule suivante :

$$E_{misfit}(\sigma,\sigma') = \alpha' \cdot \frac{\left(e_0 \cdot 10^{20}(\sigma + \sigma')\right)^2}{4 \cdot \varepsilon_0} \cdot \sqrt{\frac{\left(s_p \cdot 10^{-10}\right)^3}{\pi}}$$

Où $\alpha'$ désigne un des paramètres universels du modèle physique, comme illustré sur la figure 2.

Contribution HB

La contribution HB du potentiel chimique d'excès est calculée en utilisant la formule suivante :

$$\mu_i{}^{E, HB} = \mu_i^{HB,S} - \mu_i^{HB,ref}$$

$\mu_i^{HB,S}$ étant la contribution HB du potentiel chimique de la molécule dans la solution et

$\mu_i^{HB,ref}$ étant son potentiel chimique dans un état de référence choisi par l'utilisateur.

$\mu_i^{HB,S}$ est la somme des contributions donneur et accepteur.

$$\mu_i^{HB,S} = \int p_i^{HB}(\sigma_d) \cdot \mu^{HB,S}(\sigma_d)d\sigma_d + \int p_i^{HB}(\sigma_a) \cdot \mu^{HB,S}(\sigma_a)d\sigma_a$$

Le premier terme de cette sommation, étant la contribution donneur HB du potentiel chimique, est calculé comme étant la somme, pondérée par les nombre des segments accepteurs HB représentés dans le sigma-profile accepteurs HB, des potentiels chimiques des segments de surface donneurs HB constituant la molécule.
Le potentiel chimique du segment de surface donneur HB dépend des énergies d'interaction HB entre tous les segments de surface donneur et les segments de surface accepteur.

$$\mu^{HB,S}(\sigma_d) = -k \cdot T \cdot \ln\left(\int \overline{p^{HB,S}(\sigma_a)} \cdot exp\left(\frac{\mu^{HB,S}(\sigma_a) - E_{HB}(\sigma_a,\sigma_d)}{k \cdot T}\right) d\sigma_a\right)$$

Cette énergie d'interaction peut être calculée par la formule suivante :

$$E_{HB}(\sigma_d,\sigma_a) = c_{HB} \cdot \frac{e_0^2 \cdot (\sigma_d \cdot \sigma_a)}{4 \cdot \pi \cdot \varepsilon_0} \cdot \frac{s_p^2}{r_{da} \cdot 10^{-10}}$$

$r_{da}$ est la distance minimale d'approche entre le donneur et l'accepteur de liaison HB. $c_{HB}$ et $r_{da}$ désignent des paramètres universels du modèle physique, comme illustré sur la figure 2.

9

Le deuxième terme de cette sommation, étant la contribution accepteurs HB du potentiel chimique, est calculé comme étant la somme, pondérée par les nombre des segments donneurs HB représentés dans le sigma-profile donneurs HB, des potentiels chimiques des segments de surface accepteurs HB constituant la molécule.

Le potentiel chimique du segment de surface accepteur HB dépend aussi des énergies d'interaction HB entre tous les segments de surface donneur et les segments de surface accepteur.

$$\mu^{HB,S}(\sigma_a) = -k \cdot T \cdot \ln\left(\int \overline{p^{HB,S}(\sigma_d)} \cdot exp\left(\frac{\mu^{HB,S}(\sigma_d) - E_{HB}(\sigma_a, \sigma_d)}{k \cdot T}\right) d\sigma_d\right)$$

Les deux contributions donneur HB et accepteur HB sont interdépendantes et sont donc calculées simultanément de façon itérative.

Cette formulation de la contribution « liaison hydrogène » est entièrement nouvelle. Cette contribution est par exemple dénommée « première contribution chimique ».

Le modèle résultant prend en compte simultanément cette première contribution chimique, la contribution combinatoire et la contribution électrostatique.

Lorsqu'on dissout un carbohydrate tel que le sorbitol dans de l'eau, le carbohydrate s'hydrate une ou plusieurs fois formant ainsi des espèces hydratées *i.e.* de nouvelles espèces chimiques contenant une molécule de carbohydrate fortement liée à une ou plusieurs molécules d'eau par des liaisons hydrogènes en particulier dans la première sphère d'hydratation. Le phénomène est illustré à la figure 1 où la typologie des espèces chimiques en présence au sein d'une solution de sorbitol et d'eau (espèces apparentes) est réalisée. Les espèces vraies ainsi déterminées sont l'eau, le sorbitol anhydre, le sorbitol hydraté une fois, deux fois et trois fois. De plus, la surface colorée entourant chaque espèce vraie permet d'illustrer de façon imagée le contenu du vecteur de grandeurs physiques associé à ladite espèce vraie.

Un modèle chimique est donc nécessaire pour prendre en compte la contribution d'associations chimiques (solvatation, complexation,...) déduites des propriétés de liaisons entre les molécules composant la solution comme les interactions de nature chimique entre le sorbitol et le solvant, en particulier les réactions successives d'hydratation du sorbitol décrivant la formation chimique des formes hydratées du carbohydrate. Cette contribution est par exemple dénommée « deuxième contribution chimique ».

Ces réactions, et donc la composition des différentes espèces vraies anhydres ou hydratées présentes dans le mélange, sont caractérisées par des constantes d'équilibre d'hydratation, elles-mêmes dépendant du potentiel chimique de chacune des espèces vraies présentes dans le mélange.

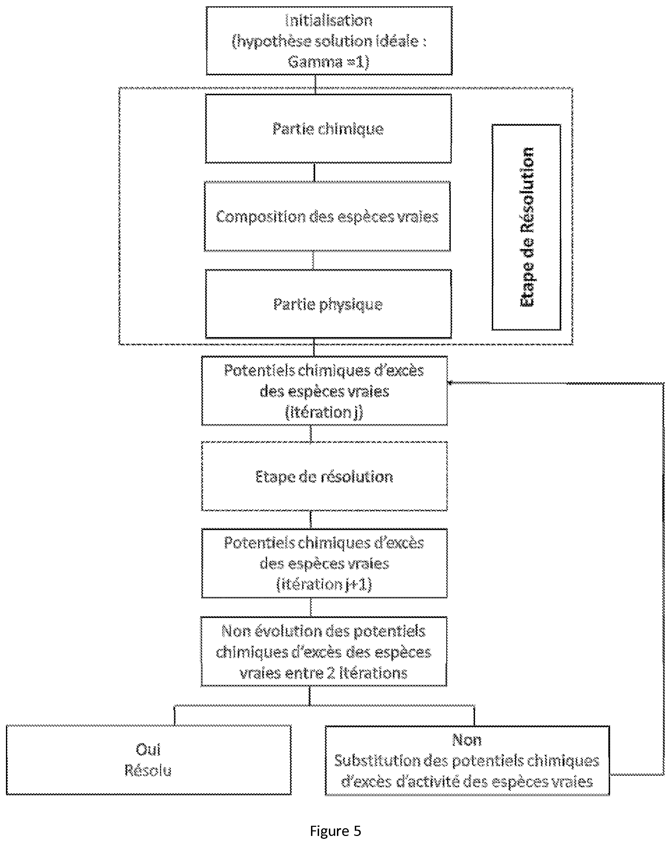

Une méthode de résolution d'équilibres physico-chimiques est donc nécessaire pour déterminer les potentiels chimiques d'excès respectifs des espèces vraies (solvant libre, espèce anhydre et espèces hydratées). Une telle méthode de résolution est illustrée à la figure 5.

A cet effet, la génération du fichier COSMO faite à la première étape du procédé doit être réalisée pour chaque espèce chimique, au sens de chaque espèce vraie, présente dans le mélange afin de rendre cohérent l'ensemble des données générées.

Cette méthode qui s'apparente à un modèle chimique permet de déterminer les propriétés d'équilibre du mélange en utilisant la méthode de résolution indiquée sur la figure 5 pour déterminer les potentiels chimiques d'excès des espèces vraies présentes dans le mélange et d'en déduire toutes les propriétés physicochimiques déduites de la connaissance de cette réalité physicochimique des interactions ayant lieu dans le mélange.

A cet effet, comme illustré sur la figure 2, l'utilisateur a simplement besoin d'indiquer les conditions (ou données) opératoires comme la composition apparente, la température et/ou la pression pour utiliser le modèle thermodynamique développé dans cette invention afin de prédire les potentiels chimiques des espèces chimiques, donc la composition des espèces vraies, présentes dans le mélange réel et les propriétés d'équilibre dudit mélange.

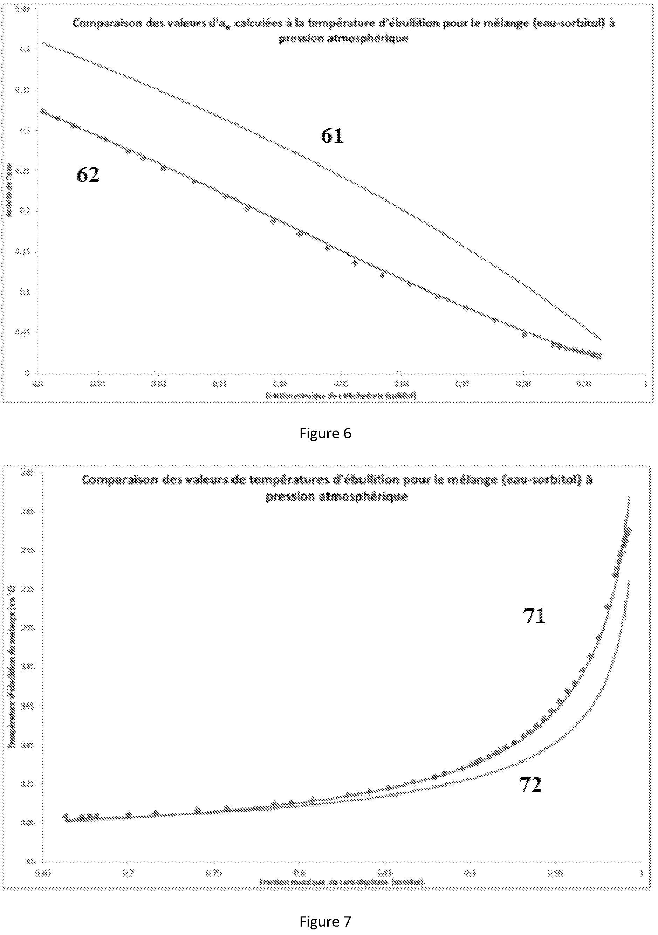

On effectue une comparaison entre les valeurs d'activité de l'eau dans une solution de sorbitol concentrée obtenues par une méthode de l'art antérieure (en l'occurrence selon le modèle de contribution de groupes UNIFAC) et la méthode de l'invention. On obtient respectivement les courbes 61 (art antérieur) et 62 (invention) de la figure 6 qui représente l'activité de l'eau en fonction de la fraction massique de sorbitol dans la solution. On y superpose des données expérimentales et on constate que l'invention permet d'obtenir des données bien plus proches des paramètres mesurés.

On effectue une comparaison entre les valeurs de températures d'ébullition à température atmosphérique dans une solution de sorbitol concentrée obtenues par une méthode de l'art antérieure (en l'occurrence selon le modèle de contribution de groupes UNIFAC) et la méthode de l'invention. On obtient respectivement les courbes 71 (art antérieur) et 72 (invention) de la figure 7 qui représente la température d'ébullition en °C en fonction de la fraction massique de sorbitol dans la solution. On y superpose des données expérimentales et on constate que l'invention permet d'obtenir des données bien plus proches des paramètres mesurés.

Dans la conception de produits finaux tels que les sorbitols commercialisés sous forme de solutions liquides concentrées, la connaissance des paramètres de contrôle d'un équipement comme un évaporateur est essentielle. Il en est de même pour les poudres de sorbitol généralement fabriquées à l'aide de solution fondue de sorbitol, i.e ayant une matière sèche très élevée, qui peut aller au-delà de 90%, voire 99%.

L'évaporation est l'étape durant laquelle on concentre des solutions diluées par transformation du solvant liquide (par exemple de l'eau) en gaz. C'est l'un des procédés les plus énergivores dans les industries chimiques et agro-alimentaires, ce qui amène les fabricants de ce type d'équipement à offrir une large gamme de technologies et systèmes pour s'adapter aux caractéristiques des produits, aux contraintes liées à la matière sèche et aux coûts énergétiques. Il est donc particulièrement avantageux de pouvoir optimiser les coûts de procédés ou simulation.

<u>Exemple : Evaluation de la technologie d'évaporation par film raclé pour la production de sorbitol fondu en continu à partir d'une solution aqueuse de sorbitol</u>

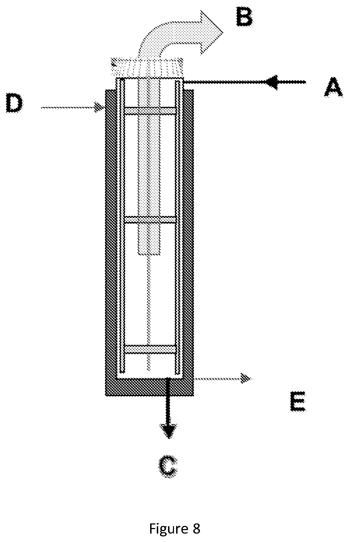

Le fonctionnement d'un évaporateur à couche mince à film raclé tel que représenté à la Figure 8 est simulé. Le flux A représente le flux de solution à évaporer (solution aqueuse de sorbitol). La vapeur de chauffe est introduite dans la double enveloppe de l'évaporateur par le Flux D. Lors de l'étape d'évaporation, l'eau évaporée de la solution de sorbitol est extraite de l'évaporateur par le Flux B. La vapeur de chauffe ainsi que les condensats de la vapeur de chauffe sont extraits de la double enveloppe par le Flux E. La solution concentrée (sorbitol fondu) est ainsi extraite de l'évaporateur par le Flux C.

Un évaporateur à couche mince à film raclé présente un haut coefficient d'échange global, ce qui permet d'évaporer très efficacement des solutions très concentrées, qui peuvent présenter une viscosité élevée.

Pour réaliser cette simulation dans laquelle les espèces apparentes sont l'eau et le sorbitol, on effectue tout d'abord la typologie des espèces vraies en présence tel qu'illustré sur la Figure 1 (eau, sorbitol anhydre, sorbitol hydraté une fois, sorbitol hydraté deux fois et sorbitol hydraté trois fois).

Des calculs quantiques sont réalisés pour chacune de ces espèces vraies à l'aide du logiciel TURBOMOLE qui permet de générer des fichiers COSMO associés à chaque espèce vraie.

De ces fichiers COSMO sont extraits les densités surfaciques de charge utilisées dans le vecteur de grandeurs physiques.

On effectue un pavage de la surface extérieure de chaque espèce vraie à l'aide de solides de Platon de type icosaèdres présentant une dimension caractéristique de 1,12 Å.

Comme illustré sur la Figure 3c pour l'eau, pour tous les segments surfaciques composant l'espèce vraie, les sigma- moléculaire 310 ($p(\sigma)$) sont générés et ceux-ci sont stockés comme valeurs dans le vecteur de grandeur physique de l'espèce vraie correspondante.

De la même manière, pour tous les segments surfaciques composant l'espèce vraie, les sigma-profile donneurs de liaison hydrogène (HB) 320 ($pHB(\sigma d)$) sont générés et ceux-ci sont stockés comme valeurs dans le vecteur de grandeur physique de l'espèce vraie correspondante.

De manière similaire, pour tous les segments surfaciques composant l'espèce vraie, les sigma-profile accepteurs de liaison hydrogène (HB) 330 ($pHB(\sigma a)$) sont générés et ceux-ci sont stockés comme valeurs dans le vecteur de grandeur physique de l'espèce vraie correspondante.

La méthode de résolution d'équilibres physico-chimiques illustrée à la figure 5 a été implantée dans le logiciel ProSimPlus (commercialisé par la société Prosim) pour déterminer les potentiels chimiques d'excès respectifs des espèces vraies (solvant libre, espèce anhydre et espèces hydratées).

Pour simuler le procédé d'évaporation, les conditions opératoires suivantes sont également rentrées dans le logiciel ProSimPlus :

- composition d'espèce apparentes de la solution à évaporer : 70% de sorbitol et 30% d'eau ;

- température d'entrée de la solution à évaporer : voir Tableau 1 ;

- débit d'entrée de la solution à évaporer : voir Tableau 1 ;

- pression absolue dans l'évaporateur : 50 mbar ;

- composition d'espèce apparentes de la solution concentrée (sorbitol fondu) : voir Tableau 1 ;

- surface d'échange de l'évaporateur : voir Tableau 1 ;

- Coefficient d'échange global de l'évaporateur : 1000 W/m2/°C.

Le modèle thermodynamique développé dans cette invention est utilisé dans ces conditions opératoires pour prédire itérativement à l'aide de ProSimPlus les potentiels chimiques d'excès des espèces apparentes, après avoir prédit les potentiels chimiques d'excès des espèces vraies présentes dans le mélange réel et les propriétés d'équilibre dudit mélange.

C'est ainsi que la température d'ébullition et le débit de sortie du mélange à la sortie de l'évaporateur sont calculés (voir tableau 1).

*Tableau 1*

| | | | 1 | 2 | 3 | 4 | 5 | 6 | 7 | 8 |
|---|---|---|---|---|---|---|---|---|---|---|
| | Echelle | | Pilote | Pilote | Industriel | Industriel | Industriel | Industriel | Industriel | Industriel |
| Données d'entrée de la simulation | Surface d'échange | m$^2$ | 0,14 | 0,14 | 3,00 | 6,00 | 6,00 | 6,00 | 6,00 | 6,00 |
| | Débit alimentation solution sorbitol | kg/h | 25 | 30 | 915 | 1500 | 1500 | 1800 | 1800 | 1800 |
| | Température de l'alimentation | (°C) | 40 | 40 | 40 | 40 | 60 | 60 | 60 | 60 |
| | Matière sèche du sorbitol fondu | % | 99,25 | 99,25 | 99,25 | 99,25 | 99,25 | 99,25 | 95,00 | 99,50 |
| Résultats de la simulation | Température de la vapeur de chauffe | (°C) | 139,5 | 146,7 | 166,9 | 154,4 | 154,4 | 167,8 | 121,1 | 173,5 |
| | Pression de la vapeur de chauffe | (bar) | 3,555 | 4,347 | 7,327 | 5,341 | 5,341 | 7,490 | 2,054 | 8,595 |
| | Température du produit concentré | (°C) | 123,7 | 123,7 | 123,7 | 123,7 | 123,7 | 123,7 | 73,0 | 130 |
| | Débit de la vapeur de chauffe | Kg/h | 10,0 | 12,8 | 390,0 | 644,9 | 644,9 | 732,9 | 530,8 | 755,7 |
| | Puissance échangée | kW | 6,37 | 7,64 | 233,04 | 382,03 | 356,33 | 427,59 | 326,13 | 437,93 |
| | DTLM (différence de température logarithmique moyenne) de l'évaporateur | (°C) | 45,5 | 54,6 | 77,7 | 63,7 | 59,4 | 71,3 | 54,4 | 73,0 |
| | Quantité eau évaporée | kg/h | 7,37 | 8,84 | 269,66 | 442,07 | 442,07 | 530,48 | 473,68 | 533,67 |
| | Débit sortie sorbitol fondu | kg/h | 17,64 | 21,16 | 645,34 | 1057,93 | 1057,93 | 1269,52 | 1326,32 | 1266,33 |

Conclusion de l'Exemple

Parmi les paramètres de contrôle déterminés par simulation grâce au procédé de l'invention, le retard à l'ébullition, i.e. la différence entre la température d'ébullition d'une solution contenant du sorbitol et celle du solvant pur (l'eau) est l'une des données les plus critiques.

De plus, la régulation de la vapeur de chauffe (débit, pression, température) est importante : elle conditionne la régularité et la stabilité de l'évaporateur.

L'utilisation de l'invention pour dimensionner (faire le design et le scale-up) est ainsi illustrée dans le Tableau 1et calibrer une installation industrielle utilisant la technologie d'évaporation par film raclé

Les simulations réalisées dans le Tableau 1 permettent ainsi de dimensionner et piloter un évaporateur à couche mince à film raclé en fonction des besoins et des utilités (vapeur, produit en alimentation, etc...) en indiquant les paramètres optimaux pour s'approcher le plus rapidement possible du point de fonctionnement.

Ces simulations permettent en effet de limiter le nombre d'essais de *scale-up* et de démarrage d'une installation industrielle.

A moins que le contraire ne soit précisé, le mot « ou » est équivalent à et/ou. De même, le mot « un » est équivalent à « au moins un » sauf si le contraire est précisé.

## Revendications

1. Méthode de détermination prédictive d'un potentiel chimique d'excès d'une espèce apparente , la méthode étant mise en œuvre par ordinateur et étant **caractérisée en ce qu'**elle comport les étapes suivantes :

   a. effectuer une typologie des espèces chimiques en présence au sein d'une solution afin de déterminer au moins une espèce vraie, et générer, pour chaque espèce vraie déterminée, un pavage de la surface moléculaire de chaque espèce chimique par une combinaison de solides de Platon de sorte que chaque espèce vraie soit associée à une forme présentant une surface extérieure constituée d'une combinaison de segments surfaciques plans et de même surface,
   b. associer à chaque segment surfacique obtenu pour une espèce vraie un vecteur de grandeurs physiques,
   c. déterminer, à partir des vecteurs de grandeurs physiques obtenus, un ensemble de contributions relatives à l'espèce vraie considérée et, à partir des contributions calculées pour les espèces vraies présentes en solution, déterminer un potentiel chimique d'excès d'une espèce apparente.

2. Méthode de détermination prédictive d'un potentiel chimique d'excès selon la revendication 1 dans laquelle la solution est une solution aqueuse de sucres et/ou de polyols et/ou de carbohydrates.

3. Méthode de détermination prédictive d'un potentiel chimique d'excès selon la revendication 1, dans laquelle la combinaison de solides de Platon est une combinaison d'icosaèdres.

4. Méthode de détermination prédictive d'un potentiel chimique d'excès selon l'une quelconque des revendications 1 à 3, dans laquelle au moins un desdits vecteurs de grandeurs physiques contient une grandeur physique choisie parmi la densité de charges, la propension à former une liaison hydrogène en tant que donneur d'atome d'hydrogène, la propension à former une liaison hydrogène en tant qu'accepteur d'atome d'hydrogène, et le potentiel électrostatique.

5. Méthode de détermination prédictive d'un potentiel chimique d'excès selon l'une quelconque des revendications 1 à 4, dans laquelle la typologie des espèces chimiques en présence distingue différents états de complexation d'une même molécule.

6. Méthode de détermination prédictive d'un potentiel chimique d'excès selon l'une quelconque des revendications 1 à 5, dans laquelle la typologie des espèces chimiques en présence distingue différents états de solvatation, en particulier d'hydratation, d'une même molécule.

7. Méthode de détermination prédictive d'un potentiel chimique d'excès selon l'une quelconque des revendications 1 à 6 comportant en outre une étape b' consistant à effectuer un histogramme moléculaire d'au moins une desdites grandeurs physiques.

8. Méthode de détermination prédictive d'un potentiel chimique d'excès selon l'une quelconque des revendications 1 à 7 comportant en outre une étape consistant à déterminer une grandeur déduite du potentiel chimique d'excès, de préférence choisie parmi un coefficient d'activité d'une espèce chimique ou d'une molécule en présence, à une température d'ébullition de la solution, et une solubilité d'une espèce chimique ou d'une molécule en présence.

9. Méthode de détermination prédictive d'un potentiel chimique d'excès selon l'une quelconque des revendications 1 à 8, dans laquelle la solution présente une teneur massique en solvant inférieure à 30% par rapport à la masse totale de la solution, avantageusement inférieure à 20%.

10. Méthode de détermination prédictive d'un potentiel chimique d'excès selon la revendication 1, dans laquelle les solides de Platon présentent une dimension caractéristique comprise entre 0.0005 Å et 100Å, de préférence entre 0.0005 Å et 6 Å.

11. Utilisation d'une méthode selon l'une quelconque des revendications 1 à 10 afin de calibrer une installation industrielle.

12. Utilisation selon la revendication 11 dans laquelle l'installation industrielle est une installation de production de sucres et/ou de polyols et/ou de carbohydrates.

13. Système informatique comprenant un processeur, ledit processeur étant configuré pour mettre en œuvre une méthode selon l'une quelconque des revendications 1 à 10 lorsqu'il reçoit des instructions spécifiques.

14. Produit-programme d'ordinateur comprenant un code configuré pour réaliser une méthode selon l'une quelconque des revendications 1 à 10 lorsqu'il est exécuté par un processeur ou une unité de contrôle électronique.

**Patentansprüche**

1. Verfahren zur prädiktiven Bestimmung eines überschüssigen chemischen Potentials einer offensichtlichen Spezies, wobei das Verfahren computergestützt durchgeführt wird und **dadurch gekennzeichnet ist, dass** es die folgenden Schritte umfasst:

a. Typologisierung der in einer Lösung vorhandenen chemischen Spezies, um wenigstens eine echte Spezies zu bestimmen, und Erzeugung einer Pflasterung der Molekülfläche jeder chemischen Spezies für jede bestimmte echte Spezies durch eine Kombination von platonischen Körpern, so dass jede echte Spezies einer Form zugeordnet ist, die eine Außenfläche aufweist, die aus einer Kombination von ebenen Flächensegmenten gleicher Fläche besteht,
b. Zuordnen eines Vektors physikalischer Größe zu jedem für eine echte Spezies erhaltenen Flächensegment,
c. Bestimmung einer Reihe von Beiträgen zu der betrachteten echten Spezies aus den erhaltenen Vektoren physikalischer Größen und auf der Basis der für die in der Lösung vorhandenen echten Spezies berechneten Beiträge Bestimmung eines überschüssigen chemischen Potentials einer offensichtlichen Spezies.

2. Verfahren zur prädiktiven Bestimmung eines überschüssigen chemischen Potentials nach Anspruch 1, wobei die Lösung eine wässrige Lösung von Zuckern und/oder Polyolen und/oder Kohlenhydraten ist.

3. Verfahren zur prädiktiven Bestimmung eines überschüssigen chemischen Potentials nach Anspruch 1, wobei die Kombination von platonischen Körpern eine Kombination von Ikosaedern ist.

4. Verfahren zur prädiktiven Bestimmung eines überschüssigen chemischen Potentials nach einem der Ansprüche 1 bis 3, wobei wenigstens einer der Vektoren physikalischer Größen eine physikalische Größe enthält, die aus der Ladungsdichte, der Neigung zur Bildung einer Wasserstoffbrücke als Wasserstoffatom-Donor, der Neigung zur Bildung einer Wasserstoffbrücke als Wasserstoff-Atomakzeptor und dem elektrostatischen Potential gewählt ist.

5. Verfahren zur prädiktiven Bestimmung eines überschüssigen chemischen Potentials nach einem der Ansprüche 1 bis 4, wobei die Typologie der vorhandenen chemischen Spezies verschiedene Komplexierungszustände desselben Moleküls unterscheidet.

6. Verfahren zur prädiktiven Bestimmung eines überschüssigen chemischen Potentials nach einem der Ansprüche 1 bis 5, wobei die Typologie der vorhandenen chemischen Spezies verschiedene Solvatationszustände, insbesondere Hydratationszustände, desselben Moleküls unterscheidet.

**7.** Verfahren zur prädiktiven Bestimmung eines überschüssigen chemischen Potentials nach einem der Ansprüche 1 bis 6, das ferner einen Schritt b' umfasst, der darin besteht, ein Molekülhistogramm wenigstens einer der genannten physikalischen Größen zu erstellen.

**8.** Verfahren zur prädiktiven Bestimmung eines überschüssigen chemischen Potentials nach einem der Ansprüche 1 bis 7, das ferner einen Schritt umfasst, der darin besteht, eine aus dem überschüssigen chemischen Potential abgeleitete Größe zu bestimmen, die bevorzugt aus einem Aktivitätskoeffizienten einer chemischen Spezies oder eines Moleküls in Gegenwart einer Lösung bei einer Siedetemperatur der Lösung und einer Löslichkeit einer vorhandenen chemischen Spezies oder eines Moleküls gewählt ist.

**9.** Verfahren zur prädiktiven Bestimmung eines überschüssigen chemischen Potentials nach einem der Ansprüche 1 bis 8, wobei die Lösung einen Massenanteil an Lösungsmittel von weniger als 30 % bezogen auf die Gesamtmasse der Lösung, bevorzugt weniger als 20 %, aufweist.

**10.** Verfahren zur prädiktiven Bestimmung eines überschüssigen chemischen Potentials nach Anspruch 1, wobei die platonischen Körper eine charakteristische Größe zwischen 0,0005 Å und 100 Å, bevorzugt zwischen 0,0005 Å und 6 Å aufweisen.

**11.** Verwendung eines Verfahrens nach einem der Ansprüche 1 bis 10 zur Kalibrierung einer Industrieanlage.

**12.** Verwendung nach Anspruch 11, wobei die Industrieanlage eine Anlage zur Herstellung von Zuckern und/oder Polyolen und/oder Kohlenhydraten ist.

**13.** Computersystem mit einem Prozessor, wobei der Prozessor dazu ausgebildet ist, ein Verfahren nach einem der Ansprüche 1 bis 10 auszuführen, wenn er bestimmte Befehle empfängt.

**14.** Computerprogrammprodukt, umfassend einen Code, der dazu ausgebildet ist, ein Verfahren nach einem der Ansprüche 1 bis 10 auszuführen, wenn er von einem Prozessor oder einer elektronischen Steuereinheit ausgeführt wird.

**Claims**

**1.** A method for the predictive determination of an excess chemical potential of an apparent species, the method being carried out by a computer and being **characterized in that** it involves the following steps:

a. performing a typology of the chemical species which are present in a solution, in order to determine at least one true species, and generating, for each true species determined, a paving of the molecular surface of each chemical species through a combination of Platonic solids, so that each true species be associated with a form having an outer surface consisting of a combination of flat surface segments of the same area
b. associating with each surface segment obtained for a true species a vector of physical magnitudes,
c. determining, from the vectors of physical magnitudes obtained, a group of contributions relative to the considered true species and, from the calculated contributions for the true species present in the solution, determining an excess chemical potential of an apparent species.

**2.** The method for the predictive determination of an excess chemical potential as claimed in claim 1, wherein the solution is an aqueous solution of sugars and/or of polyols and/or of carbohydrates.

**3.** The method for the predictive determination of an excess chemical potential as claimed in claim 1, wherein the combination of Platonic solids is a combination of icosahedra.

**4.** The method for the predictive determination of an excess chemical potential as claimed in any of claims 1 to 3, wherein at least one of said physical magnitude vectors contains a physical magnitude chosen from the charge density, the tendency to form hydrogen bonding as a hydrogen atom donor, the tendency to form hydrogen bonding as a hydrogen atom acceptor, and the electrostatic potential.

**5.** The method for the predictive determination of an excess chemical potential as claimed in any of claims 1 to 4, wherein the typology of the chemical species present enables distinction between various complexation states of the same molecule.

6. The method for the predictive determination of an excess chemical potential as claimed in any of claims 1 to 5, wherein the typology of the chemical species present enables distinction between various solvation states, in particular hydration states, of the same molecule.

7. The method for the predictive determination of an excess chemical potential as claimed in any of claims 1 to 6, further including a step b' consisting in producing a molecular histogram of at least one of said physical magnitudes.

8. The method for the predictive determination of an excess chemical potential as claimed in any of claims 1 to 7, further including a step consisting in determining a magnitude deduced from the excess chemical potential, preferably chosen from an activity coefficient of a chemical species or of a molecule present, a boiling point of the solution, and a solubility of a chemical species or of a molecule present.

9. The method for the predictive determination of an excess chemical potential as claimed in any of claims 1 to 8, wherein the solution has a solvent mass content of less than 30% relative to the total mass of the solution, advantageously less than 20%.

10. The method for the predictive determination of an excess chemical potential as claimed in claim 1, wherein the Platonic solids have a characteristic size of between 0.0005 Å and 100 Å, preferably between 0.0005 Å and 6 Å.

11. The use of a method as claimed in any of claims 1 to 10, for the calibration of an industrial facility.

12. The use as claimed in claim 11, wherein the industrial facility is a facility for producing sugars and/or polyols and/or carbohydrates.

13. A computer system comprising a processor, said processor being configured to implement a method as claimed in any of claims 1 to 10 when it receives specific instructions.

14. A computer program product comprising a code configured to implement a method as claimed in any of claims 1 to 10 when it is run by a processor or an electronic control unit.

**Espèces apparentes ou « molécules » : Eau + sorbitol**

**Espèces vraies ou « espèces chimiques » : Eau + différents états d'hydratation du sorbitol**

Figure 1

Figure 2

3a

3b

310

320

330

3c

Figure 3

Figure 4

Figure 5

Figure 6

Figure 7

Figure 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015175387 A

- WO 2012051242 A

**Littérature non-brevet citée dans la description**

- **KLAMT et al.** *J. Phys. Chem. A*, 1998, vol. 102 (26), 5074-5085
- **CATTÉ et al.** *Fluid Phase Equilibria*, 1995, vol. 105, 1-25
- Molecular thermodynamics of fluid-phase equilibria. **PRAUSNITZ et al.** Prentice-Hall international series in the physical and chemical engineering sciences. Prentice-Hall PTR., 1999, 353

- **TOURE et al.** *The Canadian Journal Of Chemical Engineering*, 2015, vol. 93, 445
- *Fluid Phase Equilibria*, 2016, vol. 424, 92
- **ACHARD et al.** *AIChE journal*, 1994, vol. 40 (7), 1210-1222
- **MOSS et al.** *Pure Applied Chemistry*, 1995, vol. 67, 1307